# EUROPÄISCHE PATENTSCHRIFT

(11) **EP 1 904 431 B1**
(45) Veröffentlichungstag und Bekanntmachung des Hinweises auf die Patenterteilung: **08.07.2015**
(21) Anmeldenummer: 06724298.2
(22) Anmeldetag: 12.04.2006
(51) Int. Cl.: C07C 53/06, C07C 51/02, A23K 1/16, A23L 1/18

(54) **HERSTELLUNG UND VERWENDUNG VON NATRIUMDIFORMIAT**
SODIUM DIFORMATE PRODUCTION AND USE
PREPARATION ET UTILISATION DE DIFORMIATE DE SODIUM

(30) Priorität: 20.02.2006 EP 06003445; 13.04.2005 DE 102005017089
(43) Veröffentlichungstag der Anmeldung: 02.04.2008
(73) Patentinhaber: BASF SE, 67056 Ludwigshafen (DE)
(72) Erfinder: HEINZ, Robert, 67067 Ludwigshafen (DE); MACHHAMMER, Otto, 68163 Mannheim (DE); GROPP, Stefan, 67346 Speyer (DE); HAUK, Alexander, 67071 Ludwigshafen (DE); DIEBOLD, Gerd, 76877 Offenbach/Queich (DE); LOHMANN, Anna, Valeska, 67117 Limburgerhof (DE); FEUERSTEIN, Dieter, 67373 Dudenhofen (DE); RÜHLE, Robert, 67316 Carlsberg (DE); SCHMITT, Rüdiger, 67063 Ludwigshafen (DE)
(86) Internationale Anmeldenummer: PCT/EP2006/003398
(87) Internationale Veröffentlichungsnummer: WO 2006/108652

(56) Entgegenhaltungen:
- WO-A-96/35657
- WO-A-03/040078
- DE-C- 424 017
- E. GROSCHUFF: "Neutrale und saure Alkaliformiate. Studien über die Löslichkeit der Salze XI." CHEMISCHE BERICHTE, Bd. 34, Nr. 147, 1903, Seiten 1783-1795, XP002387901
- GMELINS HANDBUCH DER ANORGANISCHEN CHEMIE, 8. AUFLAGE, SYSTEM-NUMMER 21: "Natrium" 1928, VERLAG CHEMIE GMBH , BERLIN , XP002387904 in der Anmeldung erwähnt Seite 816 - Seite 819 Seite 836 - Seite 841 Seite 848 - Seite 850 Seite 854 - Seite 857
- GMELINS HANDBUCH DER ANORGANISCHEN CHEMIE, 8. AUFLAGE, SYSTEM-NUMMER 22: "Kalium" 1938, VERLAG CHEMIE GMBH , BERLIN , XP002387905 Seite 919 - Seite 921 Seite 933 - Seite 935 Seite 946 - Seite 949
- E. ELÖD ET AL: 'Über das ternäre System HCOOH-HCOONa-H2O. Zur Kenntnis der sauren Natriumsalze der Ameisensäure' ZEITSCHRIFT FÜR ANORGANISCHE UND ALLGEMEINE CHEMIE Bd. 165, Nr. 1, 19 August 1927, Seiten 161 - 170, XP055105320 DOI: 10.1002/zaac.19271650117 ISSN: 0863-1786

## Beschreibung

Die vorliegende Erfindung betrifft ein Verfahren zur Herstellung von Natriumdiformiat in fester Form mit einem hohen Gehalt an Ameisensäure, dessen Verwendung in Tierfuttermitteln, als Acidifier, Konservierungsmittel, Silierhilfsmittel, Düngemittel und Wachstums- und Leistungsförderer, sowie das erfindungsgemäße Natriumdiformiat enthaltende Tierfutteradditive.

Ameisensaure Formiate besitzen eine antimikrobielle Wirkung und werden beispielsweise eingesetzt zur Konservierung sowie zur Ansäuerung von pflanzlichen und tierischen Stoffen, wie etwa von Gräsern, landwirtschaftlichen Produkten oder Fleisch, zur Behandlung von Bioabfällen oder als Additiv zur Tierernährung.

Im Bereich der Tierernährung werden als Natriumverbindungen in der Regel entweder Mischungen von Natriumdiformiat mit Trinatriumhydrogenformiat oder letzteres allein eingesetzt, siehe z. B. die WO 96/35337 und WO 04/57977. In der WO 96/35337 wird darüber hinaus über den Einsatz von Natriumdiformiat berichtet, wobei jedoch keine konkreten Angaben zur Herstellung dieser Verbindung gemacht werden.

Allgemein ist für die Verwendung von Hydrogenformiaten ein möglichst hoher Gehalt an Formiatanionen als einer der wirksamen Bestandteile wünschenswert. Aus ökonomischer Sicht ist es insbesondere vorteilhaft, wenn dieser erhöhte Gehalt an Formiatanionen mit einem möglichst hohen Anteil an Ameisensäure einher geht, da diese gleichzeitig die ansäuernde Wirkung bietet. Unter diesen Gesichtspunkten ist die Verwendung von ameisensaurem Natriumformiat besonders günstig, da bei diesem im Vergleich zu Trinatriumhydrogentetraformiat sowie gegenüber ameisensaurem Kaliumformiat jeweils ein höherer theoretischer Gehalt sowohl an Formiationen als auch an Ameisensäure vorliegt. Beide Werte sind beim Ammoniumdiformiat zwar noch etwas günstiger, jedoch handelt es sich hierbei um eine sehr unbeständige Verbindung.

Als solche sind ameisensaure Formiate in fester Form und ihre Herstellung seit langem bekannt, z. B. aus Gmelins Handbuch der anorganischen Chemie, 8. Auflage, Nummer 21, Seiten 816 bis 819, 836 bis 841 und 848 bis 857, Verlag Chemie GmbH, Berlin 1928 sowie Nummer 22, Seiten 919 bis 921, Verlag Chemie GmbH, Berlin 1937. Hiernach sollen durch Lösen von Kaliumformiat bzw. Natriumformiat in Ameisensäure und anschließendes Abkühlen die sauren Formiate Kaliumdiformiat und Natriumdiformiat prinzipiell erhältlich sein. Neben Natriumdiformiat existiert die stabilere Kristallform Trinatriumhydrogenformiat. Es wird jedoch darauf verwiesen, dass speziell Natriumdiformiat in kristalliner, trockener Form nur schwer zugänglich und darüber hinaus relativ unbeständig ist. Die Angaben in Gmelins Handbuch lassen nur den Schluss zu, dass es sich bei den dort beschriebenen Produkten nicht um reines Natriumdiformiat handelte.

Die EP 0 824 511 B1 beschreibt ein Verfahren zur Herstellung von Produkten, welche Disalze von Ameisensäure enthalten. Hierbei werden bestimmte Alkali- bzw. Ammoniumformiate, -hydroxide, -(bi)carbonate oder Ammoniak bei 40 °C bis 100 °C mit Ameisensäure vermischt, die einen Gehalt von mindestens 50 % aufweist. Das Gemisch wird anschließend gekühlt und die Disalze werden durch Filtration gewonnen. Zwar wird die Herstellung von ameisensaurem Kaliumformiat sowie von Mischungen ameisensauren Natriumformiats mit Trinatriumhydrogentetraformiat exemplarisch dargelegt, die Herstellung von festem, reinem Natriumdiformiat wird hingegen nicht gelehrt. So erlauben nämlich die für das Verfahren angegebenen Temperaturen und Konzentrationsgrenzen für die einzusetzenden (wässrigen) Kalium- bzw. Natriumformiatlösungen nur die Herstellung von Kaliumdiformiat, da sich (wässrige) Lösungen von Natriumformiat aufgrund der gegenüber Kaliumformiat geringeren Löslichkeitsgrenze nicht in den angegebenen Konzentrationen herstellen lassen. Dementsprechend erhält man zwar Kaliumdiformiat, das Natriumdiformiat liegt jedoch ausschließlich in Mischung mit dem Trinatriumhydrogentetraformiat vor.

Die WO 03/040078 beschreibt ein Verfahren zur Herstellung von ameisensauren Formiaten, bei dem man Ameisensäuremethylester mit Wasser und einer basischen Verbindung mit einem pKₐ-Wert der korrespondierenden Säure der entsprechenden Dissoziationsstufe von ≥ 3, gemessen bei 25 °C in wässriger Lösung, umgesetzt, das gebildete Methanol abtrennt sowie optional den gewünschten Säuregehalt durch Zugabe von Ameisensäure einstellt.

E. Groschuff beschreibt in Chemische Berichte, Bd. 34, Nr. 147, 1903, Seiten 1783 bis 1795 neutrale und saure Alkaliformiate und Studien über die Löslichkeit der Salze.

Die WO 96/35657 beschreibt ein Verfahren zur Herstellung von Disalzen der Aminosäure, bei dem man K-, Na- Cₛ- oder NH₄-Formiat, K-, Na- oder Cₛ-Hydroxid, K-, Na-Cₛ-Carbonat oder -Bicarbonat oder NH₃ bei 40 bis 100 °C mit Ameisensäure mischt, die Mischung kühlt und zentrifugiert.

Gmelins Handbuch der anorganischen Chemie, 8. Auflage, "Kalium", 1938, Seiten 919 bis 949 beschreibt Kaliumsalze der Ameisensäure.

Die deutsche Patentschrift DE 42 40 17 (vom 14.01.1926) lehrt die Herstellung von ameisensauren Natriumformiaten mit verschiedenem Säuregehalt durch Einbringen von Natriumformiat in wässrige Ameisensäure. Die dabei entstehenden Kristalle werden durch Abkühlung der Lösung auf Umgebungstemperatur erhalten. In Abhängigkeit vom Wassergehalt der Ameisensäure soll neben Trinatriumhydrogenformiat und Mischungen von Trinatriumhydrogenformiat mit Natriumdiformiat angeblich auch Natriumdiformiat zugänglich sein. Letzteres soll nach dem Verfahren der DE 424017 erhalten werden, wenn die eingesetzte Ameisensäure einen Gehalt von mehr als 50 % aufweist, z. B. 80 % wie in Beispiel 2. Eigene Versuche der Erfinder ergaben jedoch, dass sich unter den in der DE 424017 genannten Bedingungen kein Natriumdiformiat in reiner, kristalliner Form erhalten lässt. Vielmehr wird bei dieser Vorgehensweise ein Gemisch mit Trinatriumhydrogenformiat erhalten, dessen Gehalt an Ameisensäure deutlich unterhalb des für reines Natriumdiformiat erwarteten theoretischen Wertes von 40,36 Gew.-%, bezogen auf das Gesamttrockengewicht, liegt.

Folglich ist die Herstellung von festem Natriumdiformiat mit einem Gehalt an Ameisensäure von wenigstens 35 Gew.-% in reiner, stabiler und trockener Form bisher nicht möglich.

Eine ausreichende Stabilität von ameisensaurem Natriumformiat in fester Form ist sowohl hinsichtlich der Handhabung und Lagerfähigkeit als auch hinsichtlich der Herstellung von besonderer Bedeutung. Insbesondere ist eine in größerem Maße auftretende Freisetzung der in dem ameisensauren Natriumformiat enthaltenen Ameisensäure aufgrund deren korrosiver Wirkung unerwünscht.

Im Bereich der Tierernährung bietet Natriumdiformiat den Vorteil, dass das Spurenelement Natrium nicht wie sonst üblich in Form von NaCl gesondert zugesetzt werden muss, sondern bereits als solches eine Natriumquelle darstellt. Durch den hohen Gehalt an Ameisensäure im Natriumdiformiat, z. B. gegenüber Trinatriumhydrogentetraformiat, ist der Gehalt an Natriumionen begrenzt. Ein geringer bzw. begrenzter Gehalt an Kationen, z. B. auch Kaliumionen, ist insofern wünschenswert, als da letztere insbesondere bei Monogastriern und speziell bei Geflügel zu einer erhöhten Flüssigkeitsaufnahme (vermehrtem Trinken) und damit zu einer Verdünnung des Kots der Tiere führen, also eine diurethische Wirkung entfalten können.

Der vorliegenden Erfindung lag die Aufgabe zugrunde, ein Verfahren zur Herstellung von Natriumdiformiat in fester Form und mit hoher Reinheit, d. h. speziell mit einem hohen Gehalt an Ameisensäure, bereitzustellen. Das erfindungsgemäße Verfahren sollte insbesondere die Herstellung eines Natriumdiformiats in vergleichsweise stabiler und trockener Form ermöglichen und somit einen Weg zu einer großtechnischen Produktion eröffnen.

Diese Aufgabe wurde überraschend gelöst, indem man die Zielverbindung aus einer Mischung von Natriumformiat mit einem mehr als anderthalbfachen molaren Überschuss konzentrierter oder wässriger Ameisensäure unter Einhaltung eines molaren Verhältnisses von Ameisensäure zu Wasser von mindestens 1,1 : 1 auskristallisiert.

Ein erster Gegenstand der vorliegenden Erfindung ist daher ein Verfahren zur Herstellung von Natriumdiformiat in fester Form mit einem Gehalt an Ameisensäure von mindestens 35 Gew.-%, bezogen auf das Gesamtgewicht des Natriumdiformiats, bei dem man bei erhöhter Temperatur aus Natriumformiat und wässriger oder konzentrierter Ameisensäure mit einem Ameisensäuregehalt von wenigstens 74 Gew.-% eine homogene Mischung herstellt, die ein molares Verhältnis von HCOOH zu Na[HCOO] von mehr als 1,5 : 1 und ein molares Verhältnis von Ameisensäure zu Wasser von mindestens 1,1 : 1 aufweist, die Mischung abkühlt und die feste Phase von der Mutterlauge abtrennt.

Ein weiterer Gegenstand der Erfindung ist Natriumdiformiat in fester trockener Form mit einem Gehalt an Ameisensäure von mindestens 38 Gew.-%, bezogen auf das Gesamtgewicht des Natriumdiformiats, das im Pulverröntgendiffraktogramm Beugungsreflexe mindestens bei 6 der folgenden Netzebenenabstände d aufweist: 8,99; 7,40; 6,69; 5,12; 4,37; 3,83; 3,40; 3,10; 2,98; 2,94; 2,90; [A] (±0,04 [Å]).

Ein weiterer Gegenstand der Erfindung ist die Verwendung von Natriumdiformiat, wie zuvor definiert, als Futtermitteladditiv für Tierfutter, insbesondere für Monogastrier-Futter und speziell für Tierfutter für Schweine und/oder Geflügel.

Ein weiterer Gegenstand der Erfindung ist die Verwendung von Natriumdiformiat, wie zuvor definiert, als Acidifier.

Ein weiterer Gegenstand der Erfindung ist ein festes Futtermitteladditiv, enthaltend eine Verbindung, wie zuvor definiert, das im Wesentlichen frei von Kaliumionen ist.

Das erfindungsgemäße Verfahren ermöglicht erstmals die Herstellung eines Natriumdiformiats in fester trockener Form mit einem Gehalt an Ameisensäure von mindestens 35 Gew.-%, häufig wenigstens 36 Gew.-%, insbesondere wenigstens 37 Gew.-%, speziell wenigstens 38 Gew.-%, ganz speziell wenigstens 39 Gew.-% und noch spezieller wenigstens 40 Gew.-%, jeweils bezogen auf das Gesamtgewicht des Natriumdiformiats.

Unter einer homogenen Mischung im Sinne der vorliegenden Erfindung wird eine klare wässrige Lösung von Ameisensäure verstanden, bei der die gesamte in der Lösung enthaltene Menge an Natriumformiat in gelöster Form vorliegt. Gegebenenfalls enthält diese erfindungsgemäß hergestellte homogene Mischung Natriumdiformiat-Kristalle zum Zweck des Animpfens der Kristallisation, wie weiter unten im Detail beschrieben. Insofern umfasst der hier verwendete Begriff "homogene Mischung" nicht nur die Ameisensäure und gelöstes Natriumformiat enthaltende wässrige Lösung, sondern auch die gegebenenfalls zugesetzten Natriumdiformiat-Kristalle.

Die in der vorliegenden Erfindung zum Einsatz kommenden Ausgangsstoffe Natriumformiat und Ameisensäure sind kommerziell erhältlich und können als solche ohne Vorbehandlung verwendet werden.

Das bei Raumtemperatur als Feststoff vorliegende Natriumformiat kann z. B. als technisches Natriumformiat eingesetzt werden. Auch bei der Herstellung von Polyolen als Abfallprodukt anfallendes Natriumformiat ist zum Einsatz in der vorliegenden Erfindung geeignet. In der Regel setzt man ein Natriumformiat ein, dessen Gehalt an Na[HCOO] wenigstens 97 Gew.-%, bezogen auf das Gesamtgewicht der eingesetzten Natriumformiatquelle, beträgt. Vorzugsweise setzt man ein Natriumformiat ein, das weniger als 0,1 Gew.-% und insbesondere weniger als 0,05 Gew.-%, jeweils bezogen auf das Gesamtgewicht der eingesetzten Natriumformiatquelle, an Kaliumionen enthält.

Es ist ebenfalls möglich, das zur Reaktion mit der Ameisensäure vorgesehene Natriumformiat *in situ* herzustellen, z. B. durch Umsetzung von Natriumhydroxid, -carbonat oder Hydrogencarbonat mit Ameisensäure in konzentrierter wässriger Lösung, durch Umsetzung von Kohlenmonoxid mit flüssigem Natriumhydroxid oder durch Umsetzung von Methylformiat mit Natriumhydroxid. Bei dieser Variante kann man z. B. so vorgehen, dass man festes NaOH oder eine konzentrierte wässrige Lösung davon, gegebenenfalls unter Kühlung und/oder Rühren, in konzentrierter Ameisensäure löst. Dabei können die Verhältnisse der Ausgangsstoffe vorteilhafterweise direkt so gewählt werden, dass die Komponenten Ameisensäure, Natriumformiat und Wasser in der resultierenden Mischung bereits in den oben genannten erforderlichen molaren Verhältnissen vorliegen. Andernfalls ist in der Regel eine Neutralisation überschüssiger Ameisensäure und/oder eine Verringerung des Wassergehalts der Mischung durch übliche, dem Fachmann bekannte Verfahren, z. B. Verdampfen, Extraktion, Destillation und dergleichen, erforderlich. Im Übrigen können bei dieser Variante die für die allgemeine Verfahrensführung gemachten Angaben befolgt werden.

Erfindungsgemäß wird eine wässrige Ameisensäurelösung mit einem Ameisensäuregehalt von wenigstens 74 Gew.-% oder eine konzentrierte Ameisensäure eingesetzt. Unter einer konzentrierten Ameisensäure versteht der Fachmann eine Ameisensäurelösung mit einem Gehalt an Ameisensäure von 94 Gew.-% oder mehr, d. h. mit einem Restwassergehalt von weniger als 6 Gew.-%, jeweils bezogen auf das Gesamtgewicht der Ameisensäurelösung. Als wässrige Ameisensäure wird eine Lösung von Ameisensäure in Wasser mit einem Ameisensäuregehalt von weniger als 94 Gew.-%, bezogen auf das Gesamtgewicht der wässrigen Ameisensäurelösung. Die eingesetzte wässrige Ameisensäurelösung weist vorzugsweise eine Konzentration von wenigstens 75 Gew.-%, bevorzugt wenigstens 80 Gew.-% und besonders bevorzugt wenigstens 90 Gew.-% auf. Ganz besonders bevorzugt setzt man konzentrierte Ameisensäure mit einem Ameisensäuregehalt von wenigstens 94 Gew.-% ein. Die Konzentration der Ameisensäure oder -lösung wird vorzugsweise 99 Gew.-% nicht überschreiten und liegt besonders bevorzugt im Bereich von 80 bis 99 Gew.-% und speziell im Bereich von 94 bis 98 Gew.-%.

Vorzugsweise wird man konzentrierte oder wässrige Ameisensäure in einer Menge von mindestens 1,6 Mol, insbesondere mindestens 1,8 Mol und speziell mindestens 2,0 Mol HCOOH pro Mol Na[HCOO] einsetzen. Vorzugsweise wird das eingesetzte molare Verhältnis von HCOOH : Na[HCOO] im Bereich von 1,6 : 1 bis 3 : 1 und insbesondere im Bereich von 1,8 : 1 bis 2,5 : 1 liegen.

Außerdem wird man die jeweiligen Ausgangsstoffe vorzugsweise in solchen Mengen einsetzen, dass das molare Verhältnis von HCOOH : H₂O in der homogenen Mischung mindestens 1,5 : 1 und besonders bevorzugt mindestens 1,8 : 1 beträgt; ganz besonders bevorzugt liegt es im Bereich von 1,5 : 1 bis 10 : 1 und insbesondere im Bereich von 1,8 : 1 bis 6,1 : 1.

Die Reihenfolge des Einsatzes der Ausgangsstoffe ist von untergeordneter Bedeutung. Vorteilhafterweise erfolgt die Vermischung derart, dass eine homogene flüssige Mischung der Ausgangsstoffe in dem einzuhaltenden molaren Verhältnis erhalten wird. Erfindungsgemäß wird die homogene Mischung bei erhöhter Temperatur hergestellt. Hierunter versteht man in der Regel Temperaturen von wenigstens 30 °C, insbesondere wenigstens 40 °C und speziell wenigstens 50 °C. Die Herstellung einer solchen homogenen Mischung kann mit üblichen, dem Fachmann bekannten Vorgehensweisen erfolgen, z. B. durch Vermischen, Verrühren oder Lösen unter Anwendung erhöhter Temperatur oder durch eine kombinierte Anwendung dieser Methoden.

Zur Durchführung des erfindungsgemäßen Verfahrens geht man in der Regel so vor, dass man eine wässrige oder konzentrierte, bevorzugt konzentrierte Lösung der Ameisensäure vorlegt. Zu dieser Ameisensäurelösung wird das Natriumformiat in fester Form oder in Form einer wässrigen Lösung oder Suspension zugegeben. Die Zugabe kann portionsweise, z: B. in 2, 3, 4 oder mehr einzelnen Portionen, die der Mischung in einem vorgegebenen zeitlichen Abstand zueinander zugegeben werden, oder kontinuierlich, d. h. mit gleich bleibender, abnehmender oder zunehmender Geschwindigkeit, erfolgen. Während der Zugabe tritt in der Regel eine Temperaturerhöhung auf, so dass gegebenenfalls ein zusätzliches Erhitzen nicht erforderlich ist. Üblicherweise wird man die Temperatur der Mischung, z. B. durch Anpassung der Zugabegeschwindigkeit und/oder Kühlen bzw. Erhitzen der Mischung und/oder der zugegebenen Lösung oder Suspension, so einstellen, dass in der Mischung eine Temperatur im Bereich von 30 °C bis 80 °C und insbesondere von 40 °C bis 70 °C eingehalten wird. Vorzugsweise beträgt die Temperatur der Mischung nicht mehr als 65 °C. Erfindungswesentlich ist, dass die Kristallisation aus einer wässrigen Lösung erfolgt. Diese kann, wie unten noch weiter ausgeführt, bereits vor dem Beginn der Kristallisation mit Impfkristallen versetzt sein oder werden.

Während der Zugabe des Natriumformiats wird die Lösung oder Suspension vorteilhafterweise bewegt, z. B. gerührt. Das Bewegen wird nach Beendigung der Zugabe mindestens bis zum Erhalt einer homogenen Mischung, im Allgemeinen bis zum Ende oder Abbruch der Kristallisation fortgesetzt.

Erfindungsgemäß kann die Vermischung der Ausgangsstoffe in allen zum Zweck der Erzeugung einer homogenen flüssigen Mischung üblicherweise verwendeten Reaktoren, Kesseln, Kolben, Behältnissen und sonstigen Apparaturen, insbesondere in Rührbehältern mit innenliegenden Wärmetauscherflächen, durchgeführt werden. Diese sind dem Fachmann bekannt. Zur Vermeidung von Korrosionseffekten, z. B. bei Reaktoren oder Kesseln aus Stahl, ist es vorteilhaft, wenn die mit Ameisensäure in Kontakt kommenden Flächen und Wände mit einer säurebeständigen Schutzschicht, z. B. aus Teflon^{®}, beschichtet sind oder mit speziell säurebeständigen hochlegierten Stählen ausgekleidet sind.

Anschließend wird die Mischung, vorzugsweise unter fortgesetztem Rühren, zur Kristallisation gebracht. Dies kann z. B. durch teilweises Verdampfen oder durch Abkühlen, bevorzugt durch Abkühlen bewirkt werden. Wird die Kristallisation durch ein kontrolliertes Abdampfen der flüssigen Phase, vorzugsweise unter Vakuum, bewirkt oder eingeleitet bzw. beschleunigt, so wird man in der Regel sicherstellen, dass die molaren Verhältnisse der Komponenten in der homogenen Mischung zu Beginn und während der Kristallisation innerhalb der oben spezifizierten Bereiche liegen. Gegebenenfalls kann sich hierbei aber.das Verhältnis von HCOOH zu H₂O im Lauf der Kristallisation zu höheren molaren Verhältnissen als in der Ausgangslösung verschieben. Wird die Kristallisation durch Abkühlen bewirkt, so erfolgt dieses vorzugsweise langsam, vorteilhafterweise über einen Zeitraum von ein bis mehreren Stunden, z. B. bis zu 24 oder bis zu 12 h, insbesondere von 1 bis 15 h, speziell von 2 bis 10 h, spezieller von 3 bis 10 h und ganz speziell von 4 bis 8 h. Hierbei kristallisiert das Natriumdiformiat aus. Es hat sich als vorteilhaft erwiesen, wenn die Abkühlung mit einer Abkühlrate im Bereich von etwa 1 bis etwa 30 K/h und insbesondere etwa 2 bis etwa 20 K/h, z. B. etwa 5 bis 15 K/h, erfolgt. Um eine weitgehende Kristallisation der Zielverbindung zu erzielen, ist es von Vorteil, die Reaktionsmischung im genannten Zeitraum auf eine Temperatur von weniger als 30 °C, z. B. etwa 25, 20, 15 oder 10 °C oder weniger, insbesondere weniger als 20 °C, z. B. etwa 18 °C oder weniger oder 16 °C oder weniger, abzukühlen. In der Regel wird hierbei eine Temperatur von 0 °C, insbesondere 5 °C, speziell 10 °C und ganz speziell 15 °C nicht unterschritten.

Es hat sich als vorteilhaft erwiesen, nach Einsetzen der Kristallbildung die zunächst gebildeten Kristallkeime bzw. kleinen Kristalle durch Erhitzen z. B. auf eine Temperatur von maximal 65 °C, insbesondere im Bereich von 25 °C bis 50 °C, aufzulösen und den Kristallisationsvorgang anschließend durch erneutes, gegebenenfalls verlangsamtes Abkühlen wieder einsetzen zu lassen. Bei diesem erneuten Abkühlen liegt die Geschwindigkeitsrate üblicherweise im Bereich von etwa 0,5 bis etwa 20 K/h, z. B. bei etwa 1 bis 15 K/h, insbesondere bei etwa 2 bis 15 K/h, speziell bei etwa 5 bis 10 K/h und bevorzugt bei höchstens 25 K/h. Die Kristallisationstemperatur liegt in den oben genannten Bereichen.

Weiterhin kann es vorteilhaft sein, der Mischung bereits vorhandene, z. B. durch das erfindungsgemäße Verfahren zuvor hergestellte Kristalle von Natriumdiformiat zur Förderung des Kristallisationsvorgangs, d. h. zum Zweck des sogenannten "Animpfens", zuzugeben. Derartige Kristalle können in trockener oder feuchter Form, suspendiert in einer flüssigen, z. B. wässrigen oder ameisensauren, Phase oder einer Kombination dieser Formen zugegeben werden. Hierbei erfolgt die Zugabe in der Regel oberhalb einer Temperatur, die zu einer Kristallbildung führt, jedoch unterhalb einer Temperatur, bei der eine homogene Lösung vorliegt. Die Temperatur der Reaktionsmischung wird daher bei der Zugabe von Kristallen in der Regel 65 °C nicht überschreiten und vorzugsweise im Bereich von 25 bis 50 °C liegen. Der Kristallisationsvorgang kann dann, wie zuvor beschrieben, mit einer Abkühlrate im Bereich von etwa 0,5 bis etwa 20 K/h, z. B. etwa 1 bis 15 K/h, insbesondere etwa 2 bis 15 K/h und speziell etwa 5 bis 10 K/h, erfolgen. Die Kristallisationstemperatur liegt in den oben genannten Bereichen.

Im Anschluss an die Kristallisation trennt man das erhaltene feste Produkt von der Mutterlauge ab. Die Abtrennung der festen Phase von der Mutterlauge kann durch hierzu übliche, dem Fachmann bekannte Verfahren, z. B. Filtration oder Zentrifugation, vorzugsweise durch Zentrifugation, insbesondere unter Einsatz von Schub- oder Schälzentrifugen, vorgenommen werden. Bei dieser Abtrennung entfernt man die Mutterlauge häufig so weitgehend, dass die abgetrennte feste Phase einen Gehalt an Wasser im Bereich von 2 bis 0,2 Gew.-%, insbesondere im Bereich von 1,5 bis 0,4 Gew.-%, und speziell im Bereich von 1 bis 0,5 Gew.-%, jeweils bezogen auf das Gesamtgewicht der abgetrennten festen Phase, aufweist. Im Übrigen entfernt man die Mutterlauge häufig so weitgehend, dass die abgetrennte feste Phase einen Gehalt an Ameisensäure im Bereich von 40,5 bis 43,5 Gew.-%, insbesondere im Bereich von 41 bis 43 Gew.-%, speziell im Bereich von 41 bis 42,5 Gew.-%, und ganz speziell im Bereich von 41 bis 42,2 Gew.-%, jeweils bezogen auf das Gesamtgewicht der abgetrennten festen Phase, aufweist.

Üblicherweise wird das so gewonnene feuchte Produkt anschließend durch Trocknungsverfahren, z. B. unter Vakuum und/oder mäßigem Erhitzen, getrocknet. Hierfür einsetzbare Trockner und Trocknungsverfahren sind dem Fachmann an sich bekannt und z. B. in K. Kröll, Trockner und Trocknungsverfahren, 2. Aufl., Springer Verlag, Berlin 1978 beschrieben. Hierbei ist jedoch die relativ leichte Flüchtigkeit der im Produkt enthaltenen Ameisensäure sowie die begrenzte Temperaturstabilität des Produkts zu berücksichtigen. Es ist daher in der Regel erforderlich, die Trocknungsbedingungen innerhalb relativ enger Parameterbereiche zu kontrollieren, wie nachfolgend im einzelnen dargelegt.

Alternativ oder ergänzend kann das feuchte Produkt oder das Trockengut mit dem Fachmann bekannten Trocknungsmitteln versetzt bzw. vermischt werden. Dies ist ein übliches Vorgehen, um insbesondere die Rieselfähigkeit des erhaltenen Produkts zu verbessern. Hierfür geeignet sind z. B. Trocknungsmittel wie Kieselsäure, z. B. die Sipernat-Marken der Fa. Degussa, anorganische Alkali- und Erdalkalimetallsalze, z. B. Mg-, Ca-, Zn-, Na-, K-sulfate, -carbonate, -silikate oder -phosphate; ferner anorganische Pufferungsmittel wie Alkalimetallhydrogenphosphate, insbesondere Natrium- und Kaliumhydrogenphosphate, z. B. K₂HPO₄, KH₂PO₄ und Na₂HPO₄.

Während der Trocknung wird man das Produkt vorteilhafterweise im Trockner bewegen. Dies kann insbesondere durch mechanische Elemente, z. B. Mischerschaufeln, oder durch einen Gasstrom, z. B. ein übliches Wirbelbett, erfolgen. Besonders geeignete Trocknungsapparaturen bzw. Trockner sind z. B. Kontakttrockner, Wirbelschichttrockner und Strahlungstrockner, gegebenenfalls können Sprühtrockner eingesetzt werden. Speziell geeignet sind Schaufeltrockner, im Fall der Trocknung unter reduziertem Druck speziell Vakuumschaufeltrockner, oder mit Mischerschaufeln ausgestattete konvektive Trockner, z. B. der Fa. Forberg.

Vorzugsweise wird man die von der Mutterlauge abgetrennte feste Phase bei einer Produkttemperatur von nicht mehr als 60 °C, insbesondere nicht mehr als 55 °C, und speziell nicht mehr als 50 °C trocknen.

Vorzugsweise wird man die von der Mutterlauge abgetrennte feste Phase bei einem Druck von höchstens 10⁵ Pa trocknen. Im Fall einer Trocknung unter reduziertem Druck beträgt dieser bevorzugt höchstens 10⁴ Pa, und besonders bevorzugt höchstens 6·10³ Pa, z. B. im Bereich von 1·10³ Pa bis 6·10³ Pa. Besonders vorteilhafte Ergebnisse werden durch eine Vakuumtrocknung bei einem Druck von weniger als 4·10³ Pa erzielt, z. B. im Bereich von 1·10³ Pa bis 3,5·10³ Pa.

In einer bevorzugten Ausführungsform erfolgt eine Trocknung mittels Kontakttrocknung bei einer Wandtemperatur von höchstens 100°C, besonders bevorzugt höchstens 80 °C, und ganz besonders bevorzugt höchstens 65 °C. Hierbei kann man die Brüden durch einen Strippgasstrom, z. B. Luft oder Stickstoff, abtransportieren. Bei Anwendung eines solchen Strippgasstroms wird man üblicherweise pro Stunde solche Mengen an Gas durch die Trocknungsapparatur strömen lassen, dass diese in etwa der Größenordnung des Volumens der jeweiligen Trocknungsapparatur entsprechen. Besonders bevorzugt führt man die Kontakttrocknung bei einem Druck im Bereich von 2·10³ Pa bis etwa Normaldruck, z. B. im Bereich von 2·10³ Pa bis 1·10⁴ Pa, durch.

In einer weiteren bevorzugten Ausführungsform erfolgt eine Trocknung mittels konvektiver Trocknung bei einer Eintrittstemperatur des Trägergases im Bereich von 20 bis 120 °C, bevorzugter im Bereich von 50 bis 100 °C, besonders bevorzugt im Bereich von 60 bis 90 °C. Als Trägergas eignen sich z. B. Luft oder Stickstoff. Der Trägergasstrom kann in bekannter Weise geradeaus oder kreisförmig durch die Trocknungsapparatur geführt werden. Gegebenenfalls unterzieht man das Trägergas vor dem Trocknungsvorgang einer Konditionierung (Vortrocknung); eine solche kann selbstverständlich auch nach dem Trocknungsvorgang angeschlossen werden, so dass man das derart, z. B. mittels Kondensation oder Adsorption, getrocknete Gas erneut als Trägergas zur Produkttrocknung einsetzen kann. Üblicherweise wird man pro Stunde eine solche Menge an Gas durch die Trocknungsapparatur strömen lassen, dass diese etwa dem 100-fachen bis 10000-fachen des Volumens der jeweiligen Trocknungsapparatur entspricht.

Der nach der Trocknung im Produkt verbleibende Wassergehalt (Restwassergehalt) beträgt in der Regel nicht mehr als 0,5 Gew.-% und liegt üblicherweise im Bereich von etwa 0,5 bis 0,01 Gew.-%, bevorzugt bei höchstens 0,3 Gew.-%, bevorzugter höchstens 0,2 Gew.-%, besonders bevorzugt höchstens 0,15 Gew.-% und ganz besonders bevorzugt höchstens 0,1 Gew.-%, bezogen auf das Gesamtgewicht, bestimmt durch oxidimetrische Titration nach Karl Fischer (z. B. beschrieben in Wiland, Wasserbestimmung durch Karl-Fischer-Titration, Darmstadt, GIT, 1985).

Das erfindungsgemäße Verfahren kann kontinuierlich, batchweise oder semibatchweise durchgeführt werden. Hierbei wird das Natriumdiformiat in fester trockener Form wie beschrieben erhalten, wobei man vorteilhafterweise die Mutterlauge vollständig oder teilweise zur Herstellung der Ausgangsmischung bzw. zur Anpassung der molaren Konzentrationsverhältnisse im Eduktstrom wiederverwenden kann. Die Mutterlauge kann nach Abtrennung der Kristalle z. B. mit Natronlauge neutralisiert werden, wobei im Wesentlichen eine Natriumformiatlösung resultiert, die gegebenenfalls eingedampft bzw. erneut zur Kristallisation gebracht werden kann.

Das Natriumdiformiat in fester trockener Form wird durch das erfindungsgemäße Verfahren in hoher Reinheit erhalten und weist daher einen hohen Gehalt an Ameisensäure, in der Regel mindestens 35 Gew.-%, häufig wenigstens 36 Gew.-%, insbesondere wenigstens 37 Gew.-%, speziell wenigstens 38 Gew.-%, ganz speziell wenigstens 39 Gew.-% und noch spezieller wenigstens 40 Gew.-%, jeweils bezogen auf das Gesamtgewicht des Natriumdiformiats, auf. In der Regel wird der Gehalt an Ameisensäure im erfindungsgemäßen Natriumdiformiat nicht mehr als 41 Gew.-% und insbesondere nicht mehr als 40,5 Gew.-%, jeweils bezogen auf das Gesamtgewicht, betragen. Speziell liegt der Gehalt im Bereich von 38 bis 41 Gew.-%, spezieller im Bereich von 39 bis 41 Gew.-%, und ganz speziell im Bereich von 39 bis 40,5 Gew.-% oder im Bereich von 40 bis 41 Gew.-%, jeweils bezogen auf das Gesamtgewicht des erhältlichen Natriumdiformiats.

Hier und im Folgenden wird der Ausdruck Gesamtgewicht des Natriumdiformiats synonym mit dem Ausdruck Gesamttrockengewicht verwendet. Das Gesamttrockengewicht ist als das Gewicht des Natriumdiformiats zu verstehen, das sich durch Trocknen des Produkts unterhalb seiner Zersetzungstemperatur ergibt, z. B. durch Trocknen über einen Zeitraum von 1 h bei einer Temperatur von 35 °C und einem Druck von 50 mbar. Der Gehalt an Ameisensäure im trockenen Produkt kann in üblicher Weise, z. B. durch Titration der Ameisensäure mit einer Base, bestimmt werden. Es liegt naturgemäß ebenfalls ein hoher Gehalt an Formiatanionen im trockenen Produkt vor.

Das erfindungsgemäß hergestellte Natriumdiformiat wird typischerweise in kristalliner Form erhalten. Es wird angenommen, dass es im Wesentlichen oder vollständig der Formel Na[HCOO] • HCOOH entspricht, was jedoch nicht als Einschränkung der Erfindung zu verstehen ist. Bei etwa 65 °C ist mittels DSC (Differential Scanning Calorimetry, dynamische Differenzkalorimetrie) ein Phasenumwandlungspunkt zu beobachten. Die erfindungsgemäß erhaltene kristalline Modifikation des Natriumdiformiat lässt sich beispielsweise über Röntgenweitwinkelstreuung nachweisen. Unerwünschte Modifikationen, z. B. Trinatriumhydrogentetraformiat, können nach derselben Methode ebenfalls qualitativ detektiert werden. Die Angabe der Röntgenbeugungsreflexe erfolgt in der vorliegenden Anmeldung in Form der von der Wellenlänge der verwendeten Röntgenstrahlung unabhängigen Netzebenenabstände d [A], die sich aus dem gemessenen Beugungswinkel mittels der Bragg'schen Gleichung errechnen lassen.

In der Regel weist das Pulverröntgendiffraktogramm des erfindungsgemäßen Natriumdiformiats alle für die spezielle Kristallstruktur charakteristischen Beugungsreflexe auf. In Abhängigkeit vom Kristallinitätsgrad und der Texturierung der erhaltenen Kristalle kann es jedoch zu einer Abschwächung der Intensität der Beugungsreflexe im Pulverröntgendiffraktogramm kommen, die so weit gehen kann, dass einzelne intensitätsschwächere Beugungsreflexe im Pulverröntgendiffraktogramm nicht mehr detektierbar sind. Einzelne intensitätsschwächere Beugungsreflexe können daher fehlen oder das Intensitätsverhältnis im Pulverröntgendiffraktogramm kann verändert sein. Das Vorliegen sämtlicher der jeweils angegebenen Beugungsreflexe im Pulverröntgendiffraktogramm ist ein Indiz dafür, dass es sich um Verbindungen besonders hoher Kristallinität handelt. Es versteht sich für den Fachmann, dass das erfindungsgemäße Natriumdiformiat neben den jeweils angegebenen charakteristischen Beugungsreflexen weitere Beugungsreflexe aufweisen kann. Weiterhin weisen Gemische des erfindungsgemäßen Natriumdiformiats mit anderen kristallinen Verbindungen in der Regel zusätzliche Beugungsreflexe auf.

Das Pulverröntgendiffraktogramm des erfindungsgemäßen Natriumdiformiats ist üblicherweise durch Beugungsreflexe bei mindestens 6, insbesondere mindestens 8 und speziell mindestens 10 unter d = 8,99; 7,40; 6,69; 5,12; 4,37; 3,83; 3,40; 3,10; 2,98; 2,94; 2,90 [A] (±0,04 [A]) ausgewählten Netzebenenabständen gekennzeichnet. Weitere Beugungsreflexe werden häufig bei folgenden Netzebenenabständen beobachtet: d = 8,01; 4,89; 3,97; 3,50; 3,35; 3,25; 3,02; 2,83; 2,69; 2,44; 2,22 und/oder 2,16 [A] (±0,04 [A]). Es versteht sich für den Fachmann, dass sehr nahe beieinander liegende Netzebenenabstände einander im Pulverröntgendiffraktogramm überlagern können. Typische im Pulverröntgendiffraktogramm erhaltene relative Intensitäten sind in Tabelle 1 aufgeführt.

**Tabelle 1**

| Beugungsreflexe | d (± 0,04) [Å] | Iᵣₑₗ [%] |
|---|---|---|
| 1 | 8,99 | 100 |
| 2 | 8,01 | 14,4 |
| 3 | 7,40 | 23,2 |
| 4 | 6,69 | 91,4 |
| 5 | 5,12 | 26,2 |
| 6 | 4,89 | 17,5 |
| 7 | 4,37 | 21,6 |
| 8 | 3,97 | 11,7 |
| 9 | 3,83 | 43,6 |
| 10 | 3,50 | 18,3 |
| 11 | 3,40 | 29 |
| 12 | 3,35 | 16 |
| 13 | 3,25 | 19,9 |
| 14 | 3,10 | 39,1 |
| 15 | 3,02 | 19,9 |
| 16 | 2,98 | 49,7 |
| 17 | 2,94 | 48 |
| 18 | 2,90 | 31,5 |
| 19 | 2,83 | 18,8 |
| 20 | 2,69 | 16,7 |
| 21 | 2,44 | 16,7 |
| 22 | 2,20 | 16 |
| 23 | 2,16 | 16,7 |

Das erfindungsgemäß hergestellte Natriumdiformiat wird in der Regel in einer solchen Reinheit erhalten, dass das molare Verhältnis der Komponenten Natriumformiat und Ameisensäure üblicherweise im Bereich von 0,9 :1 bis 1,1 : 1; insbesondere im Bereich von 0,95 : 1 bis 1,05 : 1 liegt und speziell etwa 1 : 1 entspricht. Der Anteil an Natriumdiformiat im erhaltenen festen Produkt beträgt üblicherweise mindestens 97 Gew.-%. insbesondere mindestens 98 Gew.-% und speziell mindestens 99 Gew.-%, jeweils bezogen auf das Gesamtgewicht des festen Produkts. Als weitere Bestandteile kann das Produkt aufgrund von Restfeuchte oder kristallisierter Restfeuchte in der Regel bis zu 1,5 Gew.-% Ameisensäure, bis zu 1,5 Gew.-% Natriumformiat und/oder bis zu 0,5 Gew.-% Wasser enthalten, jeweils bezogen auf das Gesamtgewicht des Produkts.

Das erfindungsgemäß hergestellte Natriumdiformiat zeichnet sich durch eine vergleichsweise geringe Hygroskopizität aus, insbesondere im Vergleich zu Trinatriumhydrogentetraformiat. Außerdem ist das erfindungsgemäß erhaltene Natriumdiformiat hinreichend stabil, um eine unproblematische Handhabung und (Weiter-)Verarbeitung zu gewährleisten. Darüber hinaus beträgt der Gehalt an Kaliumionen des erfindungsgemäßen Natriumdiformiats in der Regel höchstens 1000 ppm und insbesondere höchstens 500 ppm, jeweils bezogen auf das Gesamtgewicht. Herstellungsbedingt liegt der Chloridgehalt im erfindungsgemäßen Natriumdiformiat in der Regel weniger als 1500 ppm und insbesondere weniger als 1000 ppm, jeweils bezogen auf das Gesamtgewicht.

Das erfindungsgemäße Verfahren zur Herstellung von festem, trockenem Natriumdiformiat in kristalliner, reiner und stabiler Form ermöglicht erstmals eine Übertragbarkeit der Herstellungsbedingungen auf einen großtechnischen Maßstab.

Je nach gewünschtem Anwendungszweck kann das erfindungsgemäß hergestellte Natriumdiformiat weiter verarbeitet werden, insbesondere können Pulver bestimmter Teilchengrößen erzeugt werden, die erzeugten Partikel mit Überzügen beschichtet werden und/oder Mischungen mit weiteren Zusatzstoffen hergestellt werden. Als Beispiele für Überzüge bzw. Coatingmaterialien seien Öle wie Sojaöl, Fette und Fettsäuren wie Palmitin- oder Stearinsäure oder Polymerüberzüge z. B. aus Polyalkylenen und Derivaten davon, genannt. Übliche Zusatzstoffe sind insbesondere Fließhilfsmittel und Trocknungsmittel wie die zuvor genannten, z. B. Kieselsäure etc. Zur Beschichtung übliche Verfahren sowie die dabei in Betracht kommenden Zusatzstoffe sind dem Fachmann auf dem jeweiligen Gebiet grundsätzlich bekannt, siehe z. B. DE 102 31 891 A1.

Das erhaltene feste Produkt kann vor und/oder nach dem Trocknungsschritt zerkleinert werden, z. B. mittels Mörsern, Schneidgeräten, Lochpressen und Walzenstühlen, agglomeriert werden, z. B. mittels Mischern, und/oder kompaktiert werden, z. B. mittels Pressen und Kompaktoren. Die für eine derartige Zerkleinerung eingesetzten Apparaturen sind dem Fachmann bekannt.

In einer bevorzugten Ausführungsform liegt das erfindungsgemäße Natriumdiformiat in fester Form als Kristallisatpulver oder als Granulat oder Kompaktat vor. Je nach anwendungstechnischer Anforderung weisen die Pulver, Granulate bzw. Kompaktate eine mittlere Partikelgrösse im Bereich von 1 µm bis 10000 µm, insbesondere von 10 µm bis 5000 µm und speziell von 100 µm bis 2500 µm auf.

Das erfindungsgemäße Natriumdiformiat in fester Form bzw. dieses enthaltende Zubereitungen eignen sich zur Verwendung in Futtermitteln für Tiere (Tierfuttermittel), insbesondere als Zusatz zu Tierfutter in Form von Futtermitteladditiven (Futtermittelzusatzstoffe) und speziell als Zusatz zu Prämixen für Tierfuttermittel. Prämixe sind Mischungen, die in der Regel Mineralstoffe, Vitamine, Aminosäure, Spurenelemente sowie gegebenenfalls Enzyme enthalten. Tierfuttermittel und Futtermittelzusatzstoffe, die das erfindungsgemäße Natriumdiformiat enthalten, sind besonders geeignet für Monogastrier wie Schweine, speziell Ferkel, Zuchtsauen und Mastschweine, sowie Geflügel, speziell Broiler, Legehennen, Puten, Enten, Gänse, Wachteln, Fasane und Strausse.

In Abhängigkeit von den übrigen im Futtermittel oder Futtermitteladditiv enthaltenen Stoffen bzw. Zusatzstoffen kann der Gehalt des erfindungsgemäßen Natriumdiformiats im Futtermittel bzw. Futtermitteladditiv stark variieren. Bei Futtermitteladditiven hängt der Gehalt außerdem von der Art der Formulierung ab, z. B. vom Zusatz von Hilfsstoffen wie Trockenmitteln, von einer eventuellen Beschichtung und vom Restfeuchtegehalt. Üblicherweise liegt der Gehalt an erfindungsgemäßem Natriumdiformiat im futtermitteladditiv z. B. im Bereich von 0,1 bis 99,5 Gew.-%, insbesondere von 0,5 bis 75 Gew.-% und speziell von 1 bis 50 Gew.-%, bezogen auf das Gesamttrockengewicht des Futtermitteladditivs. Das erfindungsgemäße Natriumdiformiat ist auch zur Verwendung in einem Prämix geeignet und kann hierbei in den üblichen Mengen eingesetzt, z. B. zugemischt, werden.

Insbesondere beim Einsatz in Tierfuttermittel und Futtermitteladditiven für Geflügel ist ein geringer Gehalt an Kaliumionen vorteilhaft, da Kalium in diesem Fall eine diurethische Wirkung entfalten kann. Der Einsatz des erfindungsgemäßen Natriumdiformiats zu dem vorgenannten Zweck stellt somit eine saure Natrium- und Formiatquelle dar, ohne dass notwendigerweise der Anteil an Kaliumionen erhöht ist. Ein weiterer Gegenstand der Erfindung ist daher ein festes Futtermitteladditiv, welches das erfindungsgemäße Natriumdiformiat in fester Form enthält und im Wesentlichen frei von Kaliumionen ist. Hierbei bedeutet im Wesentlichen frei von Kaliumionen, dass der Gehalt an Kaliumionen höchstens 1000 ppm und insbesondere höchstens 500 ppm, jeweils bezogen auf das Gewicht des Futtermitteladditivs, beträgt.

Tierfuttermittel werden so zusammengesetzt, dass der entsprechende Bedarf an Nährstoffen für die jeweilige Tierart optimal gedeckt wird. Im allgemeinen werden pflanzliche Futtermittelkomponenten wie Mais-, Weizen- oder Gerstenschrot, Sojavollbohnenschrot, Sojaextraktionsschrot, Leinextraktionsschrot, Rapsextraktionsschrot, Grünmehl oder Erbsenschrot als Rohproteinquellen gewählt. Um einen entsprechenden Energiegehalt des Futtermittels zu gewährleisten, werden Sojaöl oder andere tierische oder pflanzliche Fette zugegeben. Da die pflanzlichen Proteinquellen einige essentielle Aminosäuren nur in unzureichender Menge beinhalten, werden Futtermittel häufig mit Aminosäuren angereichert. Hierbei handelt es sich vor allem um Lysin und Methionin. Um die Mineralstoff- und Vitaminversorgung der Nutztiere zu gewährleisten, werden außerdem Mineralstoffe und Vitamine zugesetzt. Die Art und Menge der zugesetzten Mineralstoffe und Vitamine hängt von der Tierspezies ab und ist dem Fachmann bekannt (s. z. B. Jeroch et al., Ernährung landwirtschaftlicher Nutztiere, Ulmer, UTB). Zur Deckung des Nährstoff- und Energiebedarfs können Alleinfutter verwendet werden, die alle Nährstoffe im bedarfsdeckenden Verhältnis zueinander enthalten. Es kann das einzige Futter der Tiere bilden. Alternativ kann zu einem Körnerfutter aus Getreide ein Ergänzungsfutter gegeben werden. Hierbei handelt es sich um eiweiß-, mineralstoff- und vitaminreiche Futtermischungen, die das Futter ergänzen.

Das erfindungsgemäße Natriumdiformiat eignet sich insbesondere als sogenannter Acidifier. Unter Acidifiern werden solche Stoffe verstanden, die den pH-Wert absenken. Der Ausdruck umfasst sowohl solche Stoffe, die den pH-Wert im Substrat (z. B. Tierfutter) absenken, als auch solche, die den pH-Wert im Magen-Darm Trakt des Tieres absenken.

Das erfindungsgemäße Natriumdiformiat eignet sich insbesondere als Mittel mit leistungs- und/oder wachstumsfördernder Wirkung. In einer bevorzugten Ausführungsform wird das feste Natriumdiformiat als ein solches leistungs- und/oder wachstumsförderndes Mittel für Monogastrier, insbesondere für Schweine und/oder Geflügel eingesetzt.

Das erfindungsgemäße Natriumdiformiat eignet sich weiterhin als Konservierungsmittel, insbesondere als Konservierungsmittel für Grünfutter und/oder Tierfutter.

Das erfindungsgemäße Natriumdiformiat eignet sich des Weiteren als Hilfsmittel für pharmazeutische Zubereitungen, z. B. als Sprengmittel.

Das erfindungsgemäße Natriumdiformiat kann vorteilhafterweise bei der Herstellung von Silage eingesetzt werden. Es beschleunigt die Milchsäuregärung bzw. verhindert ein Nachgären und hemmt die Entwicklung schädlicher Hefen. Hierbei wirkt es in der zuvor für den Einsatz als Acidifier beschriebenen Weise auch regulierend auf den pH-Wert. Ein weiterer Gegenstand der Erfindung ist daher die Verwendung des erfindungsgemäßen Natriumdiformiats als Silierungmittel (Silierhilfsmittel).

Ein weiterer Gegenstand der vorliegenden Erfindung betrifft die Verwendung des erfindungsgemäßen Natriumdiformiats als Düngemittel.

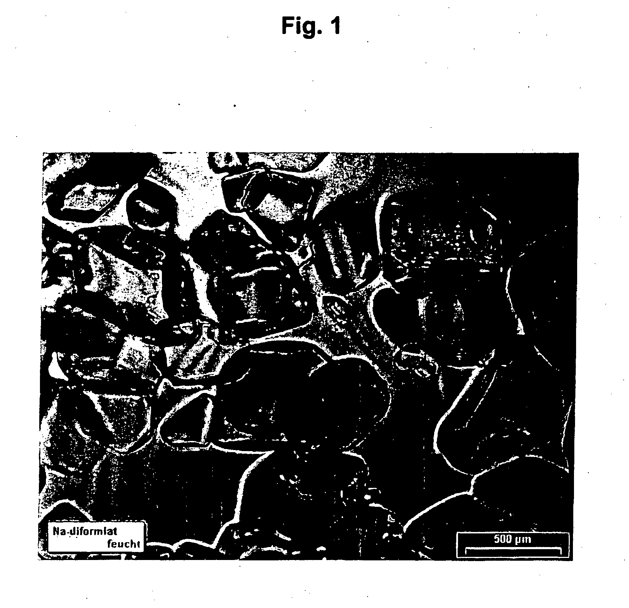

Beschreibung der.Figuren
Fig. 1 zeigt eine lichtmikroskopische Aufnahme erfindungsgemäß erhaltener Kristalle von Natriumdiformiat. Man erkennt gut die Größenverhältnisse sowie eine im Wesentlichen zumeist hexagonale Grundform.

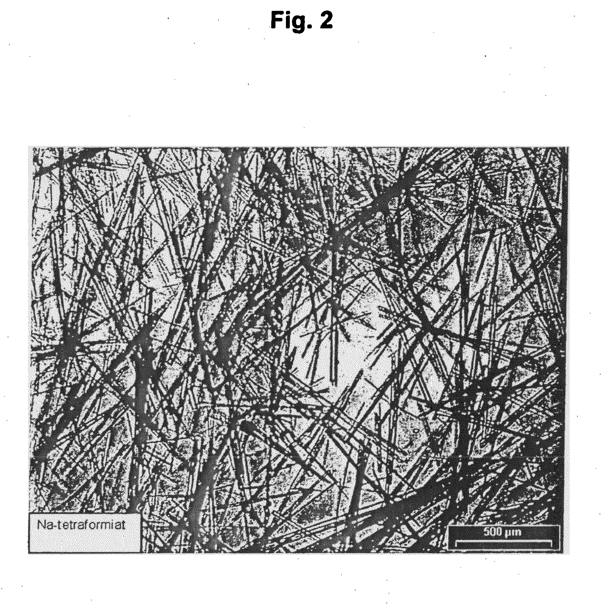

Fig. 2 zeigt zum Vergleich eine lichtmikroskopische Aufnahme von kristallisiertem Natriumtetraformiat. Man erkennt deutliche Unterschiede zu den Natriumdiformiat-Kristallen; insbesondere zeichnet sich das Natriumtetraformiat durch ein ausgeprägt nadelförmiges Kristallwachstum aus, das beim Natriumdiformiat keine Entsprechung findet. Aufgrund des relativ großen Verhältnisses von Länge zu Durchmesser erreichen die Natriumtetraformiat-Nadeln lediglich in Längsrichtung die Dimensionen der Natriumdiformiat-Kristalle von mehreren hundert bis zu über 2000 µm, während die Nadeln mit einem Durchmesser von deutlich weniger als 50 µm die Dimensionen der Natriumdiformiat-Kristalle nicht annähernd erreichen.

Die nachfolgenden Beispiele dienen zur Veranschaulichung der Erfindung und sind in keiner Weise als einschränkend zu verstehen.

### Beispiele

### Vergleichsbeispiel (analog Beispiel 2 der DE 424017)

476 g einer 80 Gew.-%igen wässrigen Ameisensäurelösung wurden vorgelegt. Unter Rühren wurden 524 g festes Natriumformiat zugegeben. Zur vollständigen Lösung wurde auf eine Temperatur von 120 °C erhitzt. Anschließend wurde die Lösung langsam abgekühlt. Ab etwa 112 °C setzte Kristallisation ein. Es wurde mit einer Rate von etwa 0,7 K/min weiter bis auf 25 °C abgekühlt. Die Suspension wurde dann 24 h unter leichtem Rühren stehen gelassen. Danach wurden die gebildeten Kristalle von der Mutterlauge abgetrennt. Die Ausbeute an feuchtem Produkt betrug etwa 370 g. Der Gehalt an Ameisensäure lag bei etwa 21,8 Gew.-%, bezogen auf das Gesamtgewicht des feuchten Produkts.

### Beispiele 1 und 2

Die Beispiele 1 und 2 wurden in einem 1 l Rührgefäß, das mit Heiz- und Kühlvorrichtung sowie einem Ablauf ausgestattet war, durchgeführt.

### Beispiel 1

650 g einer 94 Gew.-%igen wässrigen Ameisensäurelösung wurden vorgelegt und unter Rühren auf 55 °C erhitzt. Das Rühren wurde während der gesamten Versuchsdauer fortgesetzt. Es wurden 350 g festes Natriumformiat (Reinheit > 97 %) in der Ameisensäurelösung gelöst, wobei eine klare Lösung erhalten wurde. Anschließend wurde die Lösung langsam abgekühlt. Nach etwa 4 Stunden wurde eine Temperatur von etwa 12 °C erreicht, bei der ein plötzlicher Niederschlag beobachtet wurde. Die Suspension wurde auf etwa 35 °C erhitzt, bis nur noch eine leichte Trübung beobachtet wurde. Die Suspension wurde dann über einen Zeitraum von etwa 6 h auf 20 °C abgekühlt und aus dem Rührgefäß abgelassen. Die Mutterlauge wurde mittels einer Nutsche von den Kristallen abgetrennt. Die Ausbeute an feuchtem Natriumdiformiat betrug etwa 125 g. Nach der Trocknung im Vakuumtrockenschrank bei einer Temperatur von 35 °C wurde der restliche Wassergehalt im Produkt zu ca. 0,1 Gew.-%, bezogen auf das Gesamttrockengewicht von etwa 122 g, bestimmt. Der Gehalt an Ameisensäure im trockenen Produkt lag bei 40,3 Gew.-%, bezogen auf das Gesamttrockengewicht.

### Beispiel 2

650 g einer 80 Gew.-%igen wässrigen Ameisensäurelösung wurden vorgelegt und unter Rühren auf 55 °C erhitzt. Unter fortgesetztem Rühren wurden 430 g festes Natriumformiat (Reinheit > 97 %) in der Ameisensäurelösung gelöst, wobei eine klare Lösung erhalten wurde. Anschließend wurde die Lösung langsam abgekühlt. Nach etwa 5 Stunden wurde eine Temperatur von etwa 24 °C erreicht, bei der ein plötzlicher Niederschlag beobachtet wurde. Die Suspension wurde unter Rühren auf etwa 35 °C erhitzt, bis nur noch eine leichte Trübung beobachtet wurde. Die Suspension wurde dann unter Rühren über einen Zeitraum von etwa 6 h auf 15 °C abgekühlt und aus dem Rührgefäß abgelassen. Die Mutterlauge wurde mittels einer Nutsche von den Kristallen abgetrennt. Die Ausbeute an feuchtem Natriumdiformiat betrug etwa 280 g. Nach der Trocknung im Vakuumtrockenschrank bei einer Temperatur von 35 °C wurde der restliche Wassergehalt im Produkt zu ca. 0,15 Gew.-%, bezogen, auf das Gesamttrockengewicht von 270 g, bestimmt. Der Gehalt an Ameisensäure im trockenen Produkt lag bei 40,1 Gew.-%, bezogen auf das Gesamttrockengewicht.

### Beispiele 3 bis 16 (inkl. Vakuumtrocknung)

### Beispiel 3

In einem Vakuumschaufeltrockner mit 5 l Nutzvolumen (d = 134 mm, l = 413 mm, Heizfläche = 0,156 m²) wurde eine analog Beispiel 1 oder 2 erhaltene Produktmenge von 2 kg an feuchtem Kristallisat getrocknet. Die Restfeuchte vor Beginn der Trocknung betrug 0,6 Gew.-% Wasser, der Ameisensäuregehalt lag bei etwa 41 Gew.-%. Im Trockner wurden eine Wandtemperatur von 50 °C, ein Absolutdruck von 30 mbar (3000 Pa), ein Stripgasstrom von 5 Nl/h Stickstoff und eine Drehzahl von 10 min⁻¹ eingestellt. Innerhalb eines Zeitraums von weniger als 1 h wurde ein Gehalt an Restwasser von weniger als 0,1 Gew.-% erreicht. Der Gehalt an Ameisensäure betrug etwa 40,2 Gew.-%. Das erhaltene trockene Kristallisat war gut rieselfähig.

### Beispiele 4 bis 16

Die Beispiele 4 bis 16 wurden analog Beispiel 3 ausgeführt. Es wurde jeweils mit einer Drehzahl von 10 min⁻¹ gearbeitet, bei den Beispielen 14 und 16 mit einer Drehzahl von 20 min⁻¹. Die Menge des Stripgasstroms an Stickstoff betrug jeweils 5 l/h, bei Beispiel 15 abweichend 10 l/h. Weitere abweichend eingestellte Parameterwerte sind jeweils in der nachfolgenden Tabelle 2 angegeben. Tabelle 2 fasst auch die bei der Trocknung erzielten Ergebnisse zusammen.

**Tabelle 2**

| **Bsp.- Nr.** | **4** | **5** | **6** | **7** | **8** | **9** | **10** | **11** | **12** | **13** | **14** | **15** | **16** |
|---|---|---|---|---|---|---|---|---|---|---|---|---|---|
| | | | | | | | | | | | | | |
| T_{Wand} [°C] | 22 | 23 | 24 | 24 | 25 | 23 | 40 | 23 | 50 | 60 | 50 | 50 | 50 |
| T_{Prod} [°C] | 19 | 19 | 21 | 20 | 23 | 22 | 37 | 22 | 47 | 55 | 47 | 47 | 47 |
| **p_{abs} [Pa]** | 2900 | 3300 | 2700 | 2900 | 5500 | 4400 | 4500 | 5500 | 3000 | 5000 | 3000 | 3000 | 3000 |
| t [min] | 35 | 25 | 25 | 25 | 35 | 104 | 40 | 75 | 35 | 60 | 75 | 55 | 65 |
| | | | | | | | | | | | | | |
| mₜᵣ [kg] | 1,91 | 1,93 | 1,90 | 1,88 | 1,43 | 2,22 | 2,28 | 2,09 | 2,01 | 2,25 | 2,25 | 2,25 | 2,25 |
| H₂O_{Start} [Gew.-%] | 0,66 | 0,66 | 0,65 | 0,65 | 0,65 | 0.58 | 0,58 | 0,56 | 0,56 | 0,96 | 0,96 | 0,76 | 0,76 |
| H₂O_{Ende} [Gew.-%] | 0,05 | 0,06 | 0,08 | 0,07 | 0,08 | 0,12 | 0,12 | 0,12 | 0,08 | 0,09 | 0,08 | 0,10 | 0,10 |
| AS_{Start} [Gew.-%] | 41,26 | 41,26 | 41,48 | 41,48 | 41,48 | 41,17 | 41,17 | 41,28 | 41,28 | 41,66 | 41,66 | 41,42 | 41,42 |
| AS_{Ende} [Gew.-%] | 40,63 | 40,79 | 40,80 | 40,75 | 40,68 | 40,80 | 40,75 | 40,75 | 40,56 | 40,21 | 40,59 | 40,46 | 40,79 |

| | | | | | | | | | | | | | |
|---|---|---|---|---|---|---|---|---|---|---|---|---|---|
| T_{Wand} = Temperatur der Trocknerwand T_{Prod} = Produkttemperatur p_{abs} = Absolutdruck im Trockner t = Gesamtdauer des Trocknungsvorgangs mₜᵣ = Gesamttrockengewicht des erhaltenen Produkts H₂O_{Start} = Gehalt an Wasser im feuchten Produkt zu Beginn der Trocknung H₂O_{Ende} = Gehalt an Wasser im Produkt am Ende der Trocknung AS_{Start} = Gehalt an Ameisensäure im feuchten Produkt zu Beginn der Trocknung AS_{Ende} = Gehalt an Ameisensäure im Produkt am Ende der Trocknung | | | | | | | | | | | | | |

### Beispiele 17 bis 21 (inkl. konvektiver Trocknung)

### Beispiel 17

In einem gasdurchströmten Feststoffmischer mit Beckerschaufeln und 6 l Nutzvolumen (d = 210 mm, l = 180 mm) wurde eine analog Beispiel 1 oder 2 erhaltene Produktmenge von 2 kg an feuchtem Kristallisat getrocknet. Die Restfeuchte vor Beginn der Trocknung betrug 0,6 Gew.-% Wasser, der Ameisensäuregehalt lag bei etwa 41 Gew.-%. Im Trockner wurde ein Gasstrom von 5 Nm³/h Luft bzw. Stickstoff mit einer Gaseintrittstemperatur von 50 °C und eine Mischerdrehzahl von 80 min⁻¹ eingestellt. Innerhalb eines Zeitraums von weniger als 30 min wurde ein Gehalt an Restwasser von weniger als 0,1 Gew.-% erreicht. Der Gehalt an Ameisensäure betrug etwa 40 Gew.-%.

### Beispiele 18 bis 21

Die Beispiele 18 bis 21 wurden analog Beispiel 17 ausgeführt. Es wurde jeweils mit einer Drehzahl von 80 min⁻¹ gearbeitet. Die Menge des Gasstroms an Stickstoff (Beispiel 18) bzw. Luft (Beispiele 19 bis 21) betrug jeweils 5 Nm³/h, getrocknet wurde jeweils bei Normaldruck. Weitere abweichend eingestellte Parameterwerte sind jeweils in der nachfolgenden Tabelle 3 angegeben. Tabelle 3 fasst auch die bei der Trocknung erzielten Ergebnisse zusammen.

**Tabelle 3**

| **Bsp.-Nr.** | **18** | **19** | **20** | **21** |
|---|---|---|---|---|
| | | | | |
| T_{Gas} [°C] | 54 | 52 | 60 | 50 |
| T_{Wand} [°C] | 26 | 26 | 26 | 40 |
| T_{Prod} [°C] | 28,6 | 30 | 36,7 | 39,5 |
| t [min] | 70 | 60 | 65 | 60 |
| | | | | |
| mₜᵣ[kg] | 2 | 1,97 | 2,2 | 2,2 |
| H₂O_{Start} [Gew.-%] | 0,64 | 0.63 | 0,93 | 0,79 |
| H₂O_{Ende} [Gew.-%] | 0,06 | 0,06 | 0,08 | 0,06 |
| AS_{Start} [Gew.-%] | 41,52 | 41,23 | 42,05 | 41,52 |
| AS_{Ende} [Gew.-%] | 40,31 | 40,16 | 40,51 | 40,34 |

| | | | | |
|---|---|---|---|---|
| T_{Gas}= Temperatur des zur Trocknung eingeströmten Gases T_{Wand} = Temperatur der Trocknerwand I_{Prod} = Produkttemperatur t = Gesamtdauer des Trocknungsvorgangs mₜᵣ = Gesamttrockengewicht des erhaltenen Produkts H₂O_{Start} = Gehalt an Wasser im feuchten Produkt zu Beginn der Trocknung H₂O_{Ende} = Gehalt an Wasser im Produkt am Ende der Trocknung AS_{Start} = Gehalt an Ameisensäure im feuchten Produkt zu Beginn der Trocknung AS_{Ende} = Gehalt an Ameisensäure im Produkt am Ende der Trocknung | | | | |

## Patentansprüche

1. Natriumdiformiat in fester trockener Form mit einem Gehalt an Ameisensäure von mindestens 38 Gew.-%, bezogen auf das Gesamtgewicht des Natriumdiformiats, das im Pulverröntgendiffraktogramm Beugungsreflexe mindestens bei 6 der folgenden Netzebenenabstände d aufweist: 8,99; 7,40; 6,69; 5,12; 4,37; 3,83; 3,40; 3,10; 2,98; 2,94; 2,90 [A] (±0,04 [Å]).

2. Natriumdiformiat nach Anspruch 1 mit einem Gehalt an Ameisensäure im Bereich von 38 bis 41 Gew.-%, bezogen auf das Gesamtgewicht des Natriumdiformiats.

3. Natriumdiformiat nach Anspruch 1 oder 2 mit einem Wassergehalt von nicht mehr als 0,5 Gew.-%, bezogen auf das Gesamtgewicht des Natriumdiformiats.

4. Natriumdiformiat nach einem der vorherigen Ansprüche, das bei einer Temperatur von 65°C, bestimmt mittels dynamischer Differenzkalorimetrie, einen Phasenumwandlungspunkt aufweist.

5. Verfahren zur Herstellung eines Natriumdiformiats gemäß einem der Ansprüche 1 bis 4, bei dem man bei erhöhter Temperatur aus Natriumformiat und wässriger oder konzentrierter Ameisensäure mit einem Ameisensäuregehalt von wenigstens 74 Gew.-% eine homogene Mischung herstellt, die ein molares Verhältnis von HCOOH zu Na[HCOO] von mehr als 1,5 : 1 und ein molares Verhältnis von Ameisensäure zu Wasser von mindestens 1,1 : 1 aufweist, die Mischung zur Kristallisation bringt und die erhaltene feste Phase von der Mutterlauge abtrennt.

6. Verfahren nach Anspruch 5, wobei man die Mischung durch Abkühlen zur Kristallisation bringt.

7. Verfahren nach Anspruch 5 oder 6, wobei man die Mutterlauge so weitgehend entfernt, dass die abgetrennte feste Phase einen Gehalt an Wasser im Bereich von 2 bis 0,2 Gew.-%, bezogen auf das Gesamtgewicht der abgetrennten festen Phase, aufweist.

8. Verfahren nach einem der Ansprüche 5 bis 7, wobei man die Mutterlauge so weitgehend entfernt, dass die abgetrennte feste Phase einen Gehalt an Ameisensäure im Bereich von 40,5 bis 43,5 Gew.-%, bezogen auf das Gesamtgewicht der abgetrennten festen Phase, aufweist.

9. Verfahren nach einem der Ansprüche 5 bis 8, wobei man die von der Mutterlauge abgetrennte feste Phase bei einer Produkttemperatur von nicht mehr als 60°C trocknet.

10. Verfahren nach Anspruch 9, wobei die Trocknung mittels Kontakttrocknung bei einer Wandtemperatur von höchstens 100°C erfolgt.

11. Verfahren nach Anspruch 10, wobei man während der Trocknung die Brüden durch einen Strippgasstrom abtransportiert.

12. Verfahren nach Anspruch 9, wobei die Trocknung mittels konvektiver Trocknung bei einer Eintrittstemperatur des Trägergases von höchstens 100°C erfolgt.

13. Verfahren nach einem der Ansprüche 9 bis 12, wobei die Trocknung bei einem Druck von höchstens 10⁵ Pa erfolgt.

14. Verfahren nach einem der Ansprüche 9 bis 13, wobei man die feste Phase während der Trocknung mechanisch und/oder gegebenenfalls mithilfe des Trägergasstroms bewegt.

15. Verfahren nach einem der Ansprüche 9 bis 14, wobei man die Trocknung über einen solchen Zeitraum durchführt, dass das erhaltene feste Natriumdiformiat einen Gehalt an Wasser von höchstens 0,15 Gew.-%, bezogen auf das Gesamtgewicht des Natriumdiformiats, aufweist.

16. Verfahren nach Anspruch 15, wobei man die Trocknung über einen Zeitraum von nicht mehr als 120 min durchführt.

17. Verwendung von Natriumdiformiat, wie in einem der Ansprüche 1 bis 4 definiert, als Futtermitteladditiv für Tierfutter, insbesondere für Monogastrier-Futter und speziell für Tierfutter für Schweine und/oder Geflügel.

18. Verwendung von Natriumdiformiat, wie in einem der Ansprüche 1 bis 4 definiert, als Acidifier.

19. Festes Futtermitteladditiv, enthaltend eine Verbindung, wie in einem der Ansprüche 1 bis 4 definiert, das im Wesentlichen frei von Kaliumionen ist.

## Claims

1. A sodium diformate in solid dry form having a formic acid content of at least 38% by weight, based on the total weight of sodium diformate, which, in the x-ray powder diffractogram, exhibits diffraction reflections at least at 6 of the following lattice separations d: 8.99; 7.40; 6.69; 5.12; 4.37; 3.83; 3.40; 3.10; 2.98; 2.94; 2.90 [Å] (±0.04 [Å]).

2. The sodium diformate according to claim 1 having a formic acid content in the range from 38 to 41% by weight, based on the total weight of sodium diformate.

3. The sodium diformate according to claim 1 or 2 having a water content not greater than 0.5% by weight, based on the total weight of sodium diformate.

4. The sodium diformate according to one of the preceding claims which has a phase transition point at a temperature of 65°C, determined by means of differential scanning calorimetry.

5. A method for producing a sodium diformate according to one of claims 1 to 4, which comprises producing, at elevated temperature, from sodium formate and aqueous or concentrated formic acid having a formic acid content of at least 74% by weight, a homogeneous mixture which has a molar ratio of HCOOH to Na[HCOO] of greater than 1.5 : 1 and a molar ratio of formic acid to water of at least 1.1 : 1, bringing the mixture to crystallization and separating off the resultant solid phase from the mother liquor.

6. The method according to claim 5, wherein the mixture is brought to crystallization by cooling.

7. The method according to claim 5 or 6, wherein the mother liquor is removed to the extent that the solid phase which is separated off has a water content in the range from 2 to 0.2% by weight, based on the total weight of the solid phase which is separated off.

8. The method according to one of claims 5 to 7, wherein the mother liquor is removed to the extent that the solid phase which is separated off has a formic acid content in the range from 40.5 to 43.5% by weight, based on the total weight of the solid phase which is separated off.

9. The method according to one of claims 5 to 8, wherein the solid phase which is separated off from the mother liquor is dried at a product temperature of no more than 60°C.

10. The method according to claim 9, wherein the drying proceeds by means of contact drying at a wall temperature of at most 100°C.

11. The method according to claim 10, wherein, during the drying, the vapors are removed by a stripping gas stream.

12. The method according to claim 9, wherein the drying proceeds by means of convective drying at a carrier gas inlet temperature of at most 100°C.

13. The method according to one of claims 9 to 12, wherein the drying proceeds at a pressure of at most 10⁵ Pa.

14. The method according to one of claims 9 to 13, wherein the solid phase, during the drying, is agitated mechanically and/or optionally using the carrier gas stream.

15. The method according to one of claims 9 to 14, wherein the drying is carried out over such a period that the resultant solid sodium diformate has a water content of at most 0.15% by weight, based on the total weight of sodium diformate.

16. The method according to claim 15, wherein the drying is carried out over a period of not greater than 120 min.

17. The use of sodium diformate as defined in one of claims 1 to 4 as feedstuff additive for animal feed, in particular for monogastric feed, and especially for animal feed for hogs and/or poultry.

18. The use of sodium diformate as defined in one of claims 1 to 4 as acidifier.

19. A solid feedstuff additive comprising a compound as defined in one of claims 1 to 4 which is essentially free of potassium ions.

## Revendications

1. Diformiate de sodium sous forme sèche solide, ayant une teneur en acide formique d'au moins 38 % en poids, par rapport au poids total du diformiate de sodium, qui présente sur le diffractogramme de rayons X sur poudre des reflets de diffraction à au moins 6 des distances interréticulaires d suivantes : 8,99 ; 7,40, 6,69 ; 5,12 ; 4,37 ; 3,83 ; 3,40 ; 3,10 ; 2,98 ; 2,94 ; 2,90 [Å] (± 0,04 [Å]).

2. Diformiate de sodium selon la revendication 1, ayant une teneur en acide formique dans la plage allant de 38 à 41 % en poids, par rapport au poids total du diformiate de sodium.

3. Diformiate de sodium selon la revendication 1 ou 2, ayant une teneur en eau inférieure ou égale à 0,5 % en poids, par rapport au poids total du diformiate de sodium.

4. Diformiate de sodium selon l'une quelconque des revendications précédentes, qui présente un point de transformation de phases à une température de 65 °C, déterminée par calorimétrie différentielle dynamique.

5. Procédé de fabrication d'un diformiate de sodium selon l'une quelconque des revendications 1 à 4, dans lequel un mélange homogène est fabriqué à température élevée à partir de formiate de sodium et d'acide formique aqueux ou concentré ayant une teneur en acide formique d'au moins 74 % en poids, ledit mélange présentant un rapport molaire entre HCOOH et Na[HCOO] de plus de 1,5:1 et un rapport molaire entre l'acide formique et l'eau d'au moins 1,1:1, le mélange est cristallisé et la phase solide obtenue est séparée de la liqueur mère.

6. Procédé selon la revendication 5, dans lequel le mélange est cristallisé par refroidissement.

7. Procédé selon la revendication 5 ou 6, dans lequel la liqueur mère est éliminée jusqu'à ce que la phase solide séparée présente une teneur en eau dans la plage allant de 2 à 0,2 % en poids, par rapport au poids total de la phase solide séparée.

8. Procédé selon l'une quelconque des revendications 5 à 7, dans lequel la liqueur mère est éliminée jusqu'à ce que la phase solide séparée présente une teneur en acide formique dans la plage allant de 40,5 à 43,5 % en poids, par rapport au poids total de la phase solide séparée.

9. Procédé selon l'une quelconque des revendications 5 à 8, dans lequel la phase solide séparée de la liqueur mère est séchée à une température de produit inférieure ou égale à 60 °C.

10. Procédé selon la revendication 9, dans lequel le séchage a lieu par séchage par contact à une température de paroi d'au plus 100 °C.

11. Procédé selon la revendication 10, dans lequel les vapeurs sont évacuées par un courant de gaz d'extraction pendant le séchage.

12. Procédé selon la revendication 9, dans lequel le séchage a lieu par séchage convectif à une température d'entrée du gaz vecteur d'au plus 100 °C.

13. Procédé selon l'une quelconque des revendications 9 à 12, dans lequel le séchage a lieu à une pression d'au plus 10⁵ Pa.

14. Procédé selon l'une quelconque des revendications 9 à 13, dans lequel la phase solide est déplacée mécaniquement et/ou éventuellement à l'aide du courant de gaz vecteur pendant le séchage.

15. Procédé selon l'une quelconque des revendications 9 à 14, dans lequel le séchage est réalisée pendant une durée telle que le diformiate de sodium solide obtenu présente une teneur en eau d'au plus 0,15 % en poids, par rapport au poids total du diformiate de sodium.

16. Procédé selon la revendication 15, dans lequel le séchage est réalisé pendant une durée inférieure ou égale à 120 minutes.

17. Utilisation de diformiate de sodium, tel que défini dans l'une quelconque des revendications 1 à 4, en tant qu'additif d'aliments pour animaux pour l'alimentation animale, notamment d'aliments pour animaux monogastriques et particulièrement d'aliments pour porcs et/ou volailles.

18. Utilisation de diformiate de sodium, tel que défini dans l'une quelconque des revendications 1 à 4, en tant qu'acidifiant.

19. Additif solide d'aliments pour animaux, contenant un composé tel que défini dans l'une quelconque des revendications 1 à 4, qui est essentiellement exempt d'ions potassium.
